# EUROPÄISCHE PATENTANMELDUNG

(11) **EP 0 712 637 A1**
(43) Veröffentlichungstag der Anmeldung: **22.05.1996**
(21) Anmeldenummer: 95117250.1
(22) Anmeldetag: 02.11.1995
(51) Int. Cl.: A61M 15/00

(54) **Abscheidesystem für einen Pulverinhalator**

(30) Priorität: 15.11.1994 DE 4440734
(71) Anmelder: BAYER AG, 51368 Leverkusen (DE)
(72) Erfinder: Herold, Heiko, D-41470 Neuss (DE); Wollenschläger, Axel, Dr., D-51467 Bergisch Gladbach (DE)

(57) **Zusammenfassung**

Das Abscheidesystem besteht aus einem auf den Pulverinhalator aufsteckbaren Sammelrohr (1) mit einem Mundstück (3) und einem im Sammelrohr (1) angeordneten Fliehkraftabscheider (2, 6). Dieser Fliehkraftabscheider weist mindestens eine Drallerzeugerfläche (2) auf, die im Sammelrohr (1) eine Drallströmung erzeugt, wodurch die schweren Grobstaubpartikel an der Innenwand des Sammelrohrs (1) abgeschieden werden, während die leichteren Feinstaubpartikel durch eine im wesentlichen auf die Umgebung der Rohrachse beschränkte Strömung ins Mundstück (3) gelangen.

## Beschreibung

Die Erfindung betrifft ein Grobabscheidesystem, welches zusätzlich mit einem geeigneten Pulverinhalator zur Therapie mit Aerosolen eingesetzt werden kann.

Zahlreiche Arzneimittel werden in Form von Aerosolen appliziert. In der Vergangenheit wurden hierfür hauptsächlich Druckgas-Aerosole eingesetzt. Wegen der bekannten Problematik der umweltschädigenden Treibmittel gewann die Therapie mit Pulver-Aerosolen zunehmend an Bedeutung.

Bei dieser Arzneiform wird der Wirkstoff in geeigneter Formulierung, z.B. in reiner Form als weiche Pellets oder aber im Gemisch mit geeigneten Hilfsstoffen, z.B. Lactose-Monohydrat, eingesetzt.

Bei der Inhalation des Patienten wird die Formulierung in den Atemluftstrom dispergiert, wobei nur ausreichend feine Partikel den Wirkort Lunge erreichen, während gröbere Agglomerate und Partikel in den oberen Atemwegen sowie im Rachenraum abgeschieden werden.

Ein wichtiges Ziel der Entwicklung von Pulverinhalatoren und Formulierungen ist deshalb, den Feinstaubanteil zu maximieren und den Grobstaubanteil zu minimieren.

Die für die Dispergierung der Formulierung erforderliche Energie wird bei Pulverinhalatoren üblicherweise aus dem Atemluftstrom des Patienten gewonnen. Diese nur in begrenztem Umfang bereitstehende Energie reicht erfahrungsgemäß nicht für eine quantitative Dispergierung in respirable Wirkstoffpartikel aus.

Außerdem enthalten zahlreiche Aerosol-Formulierungen (z.B. adhäsive Pulvermischungen) Hilfsstoffe (z.B. Laktose Monohydrat), die von ihrer Korngröße her überwiegend als Grobstaub in den oberen Atemwegen abgeschieden werden. Die an diesen Hilfsstoffen anhaftenden feineren Wirkstoffpartikel werden so ebenfalls in den oberen Atemwegen abgeschieden.

Dies ist besonders dann von Nachteil, wenn der Wirkstoff - wie z.B. bei Corticoiden - eine unerwünschte lokale Wirkung zeigt. So wurden in der Vergangenheit häufig lokale Pilzinfektionen als Folge der Aerosol-Therapie mit Corticoiden beschrieben.

Bei den Druckgas-Aerosolen wurden zur Verringerung dieser Wirkstoffabscheidung im Rachenraum zahlreiche als "Spacer" bezeichnete Abscheidesysteme beschrieben.

Diese Abscheidesysteme bergen eine Reihe von Vorzügen:
Zum ersten wird die Geschwindigkeit des austretenden Sprühstrahles aus den Sprühköpfen verringert, wodurch die Abscheidung durch Inpaktion im Rachenraum vermindert wird.

Außerdem kann insbesondere bei großvolumigen Spacern eine quantitative Verdunstung des Treibmittels erreicht werden, wodurch sich die Tröpfchengröße der Aerosoltröpfchen verringert. Schließlich besteht die Möglichkeit, bei großvolumigen Spacern die Inhalation des Patienten von der Freisetzung des Wirkstoffes abzukoppeln und somit die Koordinationsprobleme zahlreicher Patienten zu verringern.

Während derartige Spacer für Druckgas-Aerosole allgemein in Gebrauch sind, finden sie bei Pulver-Aerosolen bislang keinen Einsatz. Dies ist insofern erstaunlich, als die prinzipielle Problematik der Abscheidung nennenswerter Wirkstoffmengen im Rachen ebenfalls bei Pulver-Aerosolen besteht

Abscheidesysteme, die in Zusammenhang mit Pulver-Aerosolen eingesetzt werden sollen, müssen allerdings andere konstruktive Eigenschaften aufweisen als Abscheidesysteme, wie sie für Druckgas-Aerosole in Gebrauch sind.

Der Erfindung liegt die Aufgabe zugrunde, ein für die Kombination mit Pulverinhalatoren geeignetes Abscheidesystem zu entwickeln, das zu einer nennenswerten Reduktion der Deposition von nicht respirablem Wirkstoff und Hilfsstoff im Rachenraum führt. Auf der anderen Seite soll die erwünschte, respirable Wirkstoffmenge durch das Abscheidesystem nicht beeinträchtigt werden.

Diese Aufgabe wird erfindungsgemäß dadurch gelöst, daß das Abscheidesystem aus einem auf den Pulverinhalator aufsteckbaren Sammelrohr mit einem Mundstück und einem im Sammelrohr angeordneten Fliehkraftabscheider besteht, der mindestens eine Drallerzeugerfläche aufweist, die im Sammelrohr eine Drallströmung erzeugt, wodurch die schweren Grobstaubpartikel an der Innenwand des Sammelrohrs abgeschieden werden, während die leichteren Feinstaubpartikel durch eine im wesentlichen auf die Umgebung der Rohrachse beschränkte Strömung ins Mundstück gelangen.

Vorteilhaft liegt der Strömungswiderstand des Fliehkraftabscheiders bei einem Inhalationsstrom von 60 l/min zwischen 0,3 mbar und 2 mbar.

Das Sammelrohr weist vorteilhaft ein Volumen zwischen 10 cm³ und 300 cm³, vorzugsweise zwischen 20 cm³ und 100 cm³, auf.

Die Drallerzeugerfläche kann aus einer ebenen oder gekrümmten, im Sammelrohr angebrachten Schaufelfläche bestehen. Bevorzugt bestehen die Drallerzeugerflächen aber aus elliptischen, den gesamten Sammelrohrquerschnitt ausfüllenden Segmenten, die in einem spitzen Winkel α zur Rohrachse geneigt sind und deren Peripherie bündig mit der Sammelrohrinnenwand abschließt.

Besonders gute Abscheideergebnisse werden erzielt, wenn die drallerzeugenden Segmentflächen so gestaltet sind, daß an den Innenkanten der Segmente ein zentraler Durchbruch in Rohrmitte verbleibt.

Der Winkel α zwischen der Rohrachse und den Segmentflächen liegt vorzugsweise im Bereich von 40° bis 70°. Das optimale Verhältnis von Innendurchmesser des Sammelrohres zum Durchmesser des zentralen Durchbruchs liegt im Bereich von 2 bis 10.

Das hier beschriebene System zeichnet sich besonders durch folgende Eigenschaften aus:
Im Vergleich mit Abscheidesystemen nach dem Prinzip der Prallabscheidung zeigt das hier beschriebene System beim Gebrauch keinen nennenswerten eigenen Druckverlust auf. Hierdurch wird vermieden, daß sich der Volumenstrom im Inhalator bei der Anwendung reduziert, was nachteiligen Einfluß auf die Dispergierung der Formulierung und die Dosiergenauigkeit haben würde. Außerdem wird der für den Patienten unangenehme Strömungswiderstand des Gesamtsystems nicht merkbar erhöht.

Eine weitere Eigenschaft des vorgestellten Systems ist, daß es sich durch ein geringes Volumen auszeichnet, wodurch es sich insbesondere von Spacern, wie sie bei Druckgas-Aerosolen gebräuchlich sind, unterscheidet. Hierdurch wird verhindert, daß ein nennenswerter Teil des vom Patienten inhalierten Luftstromes nicht mit Wirkstoff befrachtet ist. Außerdem ermöglicht die hierdurch kleine Bauform des Gerätes eine mühelose Mitnahme durch den Patienten.

Ein weiterer Vorteil des hier vorgestellten Systems besteht darin, daß es sich vom Patienten auf einfache Weise zerlegen und reinigen läßt.

Schließlich zeigt ein System nach dem hier vorgestellten Prinzip keine nennenswerte Reduktion des gewünschten respirablen Feinstaubs, wenn das Abscheidesystem zwischen Pulverinhalator und Mund des Patienten eingesetzt wird, während Systeme nach dem Prallabscheideprinzip teilweise auch den gewünschten respirablen Feinstaub mit abscheiden, wodurch die die Lunge erreichende Wirkstoffdosis in unerwünschter Weise reduziert wird.

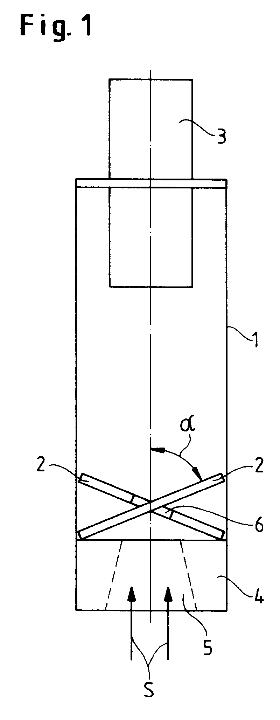

Im folgenden wird die Erfindung anhand eines in den Zeichnungen dargestellten Ausführungsbeispieles näher erläutert. Es zeigen:
- Fig. 1: einen Aufriß bzw. eine Seitenansicht des Abscheidesystems
- Fig. 2: einen Längsschnitt durch das Abscheidesystem
- Fig. 3: einen Querschnitt A-A durch das Abscheidesystem gemäß Fig. 2
Das Abscheidesystem gemäß Fig. 1 besteht aus einem Sammelrohr 1 mit eingebauten Drallerzeugerflächen 2, einem Mundstückrohr 3 an dem einen Ende und einem Adapter 4 an dem anderen Ende. Der Adapter 4 ist mit einer abgeflachten, kegelstumpfähnlichen Öffnung 5 versehen. Die Öffnung 5 ist in ihrer Form an das eigentliche Mundstück eines Pulverinhalators angepaßt, so daß das Abscheidesystem mit der Öffnung 5 auf den Pulverinhalator aufgesteckt werden kann. Das Mundstückrohr 3, das mit einem Teil seiner Länge in das Sammelrohr 1 hineinragt, bildet dann das neue Mundstück für das aus dem Pulverinhalator und dem Abscheider bestehende Inhalatorsystem.

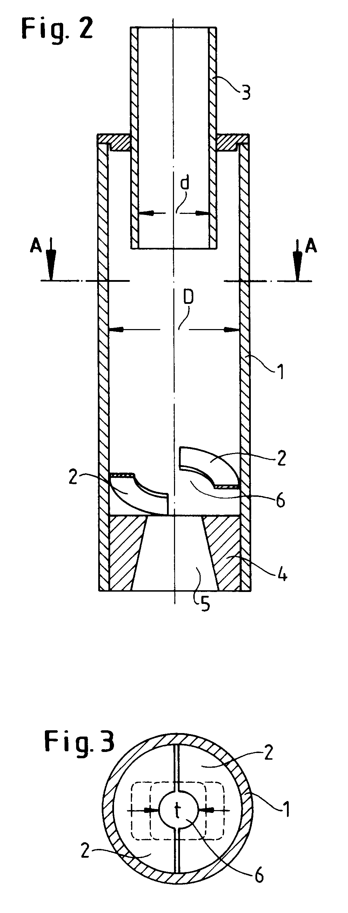

Die Drallerzeugerflächen 2 sind in dem Sammelrohr 1 in Schräglage eingebaut und bestehen aus elliptischen, den gesamten Sammelrohrquerschnitt ausfüllenden Segmenten (siehe Fig. 2). Die Segmente bilden mit der Rohrachse einen spitzen Winkel α, der zwischen 40° und 70° liegt (siehe Fig. 1). Die Segmente 2 sind ferner an ihren Innenkanten, d.h. in Rohrmitte, mit einem zentralen kreisförmigen Durchbruch 6 versehen.

Die aus den elliptischen Segmenten 2 bestehenden Drallerzeugerflächen erteilen der vorher im wesentlichen achsenparallelen Strömung (Strömungspfeile S) eine Drallkomponente, die eine spiralförmige Strömung im Sammelrohr 1 bewirkt. Dies hat zur Folge, daß die relativ schwereren pulverförmigen Teilchen aufgrund der Zentrifugalkraft an die Sammelrohrinnenwand geschleudert werden, während die leichteren Feinstaubpartikel durch eine im wesentlichen auf die Umgebung der Rohrachse beschränkte Strömung ins Mundstückrohr 3 gelangen. Die schweren Grobstaubpartikel werden also im Sammelrohr 1 abgeschieden, das von Zeit zu Zeit ausgeleert werden kann. Die klassierende Wirkung des Abscheiders wird durch den zentralen Durchbruch 6 an der Innenseite der elliptischen Segmente 2 noch weiter verbessert, weil dort keine Prallabscheidung des Feinstaubs stattfinden kann. Vor allem wird aber dadurch der Strömungswiderstand des Abscheidesystems günstig beeinflußt (erniedrigt). Besonders günstige Strömungsbedingungen für die Inhalation kann man erreichen, wenn der Strömungswiderstand des Fliehkraftabscheiders bei einem Inhalationsstrom von 60 l/min zwischen 0,3 und 2 mbar beträgt. Zu diesem Zweck müssen im wesentlichen folgende Dimensionierungsvorschriften eingehalten werden:
- Volumen des Sammelrohrs 1 zwischen 20 cm³ und 100 cm³
- Neigungswinkel α der elliptischen Segmente 2 zwischen 40° und 70°
- Verhältnis des Innendurchmessers D des Sammelrohrs 1 zum Durchmesser t des zentralen Durchbruchs 6 zwischen 2 und 10.

### Ausführungsbeispiel

Für die Grobgutabscheidung eines staubförmigen Aerosols wurde einem Pulverinhalator ein Abscheidesystem mit einem Innendurchmesser D des Sammelrohres von 24 mm nachgeschaltet. Das Volumen des Abscheidesystems lag bei 30 cm³. Die Drallerzeugung erfolgte durch zwei ebene Schaufeln, die in einem Winkel von 60° zur Rohrachse geneigt waren und deren Peripherie bündig mit der Innenwand des Sammelrohres abschlossen. In der Rohrmitte verblieb ein zentraler Durchbruch. Das Verhältnis vom Innendurchmesser des Sammelrohres zu dem des zentralen Durchbruchs lag bei 3,6. Der Druckverlust des Abscheidesystems wurde bei einem Inhalationsstrom von 60 l/min mit 0,9 mbar gemessen.

Bei den Inhalationsvorgängen gelang es, den das Abscheidesystem verlassenden Grobstaubanteil im Vergleich zum Eintritt auf weniger als 40 % zu reduzieren, wobei der Feinanteil nahezu völlig konstant blieb.

## Patentansprüche

1. Abscheidesystem für einen Pulverinhalator, dadurch gekennzeichnet, daß das Abscheidesystem aus einem auf den Pulverinhalator aufsteckbaren Sammelrohr (1) mit einem Mundstück (3) und einem im Sammelrohr (1) angeordneten Fliehkraftabscheider (2, 6) besteht, der mindestens eine Drallerzeugerfläche (2) aufweist, die im Sammelrohr (1) eine Drallströmung erzeugt, wodurch die schwereren Grobstaubpartikel an der Innenwand des Sammelrohrs (1) abgeschieden werden, während die leichteren Feinstaubpartikel durch eine im wesentlichen auf die Umgebung der Rohrachse beschränkte Strömung ins Mundstück (3) gelangen.

2. Abscheidesystem nach Anspruch 1, dadurch gekennzeichnet, daß der Strömungswiderstand des Fliehkraftabscheiders bei einem Inhalationsstrom von 60 l/min zwischen 0,3 mbar und 2 mbar liegt.

3. Abscheidesystem nach Ansprüchen 1 und 2, dadurch gekennzeichnet, daß das Sammelrohr (1) ein Volumen zwischen 10 cm³ und 300 cm³, vorzugsweise zwischen 20 cm³ und 100 cm³, hat.

4. Abscheidesystem nach Ansprüchen 1 bis 3, dadurch gekennzeichnet, daß die Drallerzeugerfläche aus einer ebenen oder gekrümmten Schaufelfläche besteht.

5. Abscheidesystem nach Ansprüchen 1 bis 3, dadurch gekennzeichnet, daß die Drallerzeugerflächen aus elliptischen, den gesamten Sammelrohrquerschnitt ausfüllenden Segmenten (2) bestehen, die in einem spitzen Winkel α zur Rohrachse geneigt sind und deren Peripherie bündig mit der Sammelrohrinnenwand abschließt.

6. Abscheidesystem nach Anspruch 5, dadurch gekennzeichnet, daß an den Innenkanten der Segmente (2) ein zentraler Durchbruch (6) in Rohrmitte verbleibt.

7. Abscheidesystem nach Ansprüchen 5 und 6 , dadurch gekennzeichnet, daß der Winkel α im Bereich von 40° bis 70° liegt.

8. Abscheidesystem nach Anspruch 6, dadurch gekennzeichnet, daß das Verhältnis von Innendurchmesser D des Sammelrohres (1) zum Durchmesser t des zentralen Durchbruchs (6) 2 bis 10 beträgt.
